# EUROPEAN PATENT APPLICATION

(11) **EP 2 161 266 A1**
(43) Date of publication of application: **10.03.2010**
(21) Application number: 08162823.2
(22) Date of filing: 22.08.2008
(51) Int. Cl.: C07D 407/12, C07D 307/85, C07D 405/12, C07D 413/12, A61P 25/00, A61K 31/343

(54) **Benzofuran derivatives as orexin receptor antagonists**

(71) Applicant: EVOTEC Neurosciences GmbH, 22525 Hamburg (DE)
(72) Inventor: Bentley, Jonathan Mark, Abingdon OX14 4SA (GB); Vaidya, Darshan Gunvant, Abingdon OX14 4SA (GB); Heifetz, Alexander, Abingdon OX14 4SA (GB); Slack, Mark, 22527 Hamburg (DE)
(74) Representative: Büchel, Edwin

(57) **Abstract**

The invention relates to compounds of formula (I) 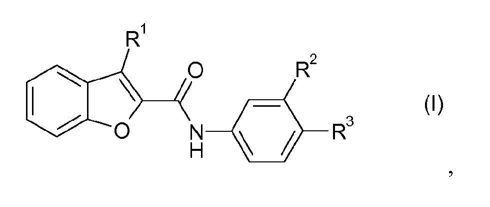 wherein R¹, R² and R³ have the meaning as cited in the description and the claims. Said compounds are useful as Orexin Receptor antagonists. The invention also relates to pharmaceutical compositions, the preparation of such compounds as well as the production and use as medicament.

## Description

The present invention relates to Orexin Receptor antagonists, especially Orexin 1 Receptor antagonists, pharmaceutical compositions thereof, the preparation of such compounds as well as the production and use as medicament.

The orexins (orexin A or OX-A and orexin B or OX-B) are neuropeptides discovered in 1998 by two research groups; orexin A is a 33 amino acid peptide and orexin B is a 28 amino acid peptide which are derived from a common pre-propeptide (Sakurai et al., Cell, 1998, 92, 573-585; De Lecea L. et al, PNAS, 1998, 95, 322-27). Orexins are produced in the lateral hypothalamus and bind to two eponymous G-protein-coupled receptors (OX1 and OX2). The OX1 receptor is selective for OX-A, whereas the OX2 receptor binds both OX-A and OX-B.

It is believed that Orexin Receptors are associated with diseases and disorders including eating disorders, sleep disorders, cognitive dysfunctions in psychiatric and neurological disorders, drug dependence, obesity, and Type II Diabetes.

Orexin Receptor antagonists are described in the art. However, most of these compounds are non-selective and used as dual, OX1 and OX2 receptor, antagonists.

For example, cinnamide compounds as antagonists are described in WO-A 00/47576. Phenylurea compounds are described in WO-A 00/47577. Tetrahydroisoquinoline derivatives are decribed in, e.g., WO-A 01/68609. Piperidines are described in WO-A 01/96302. N-Aroyl cyclic amine derivatives are described in, e.g., WO-A 02/089800. 7,8,9,10-Tetrahydro-6H-azepino-, 6,7,8,9-tetrahydro-pyrido- and 2,3-dihydro-2H-pyrrolo[2,1-b]-quinazolinone derivatives are described in WO-A 2004/004733. Azetidine compounds are described in WO-A 2008/020405.

Benzofuran derivatives useful in the medical field are described in, e.g., JP-A 50-035310 and JP-A 50-049270, where 3-methyl-2-benzofuran-carboxamides are used, including N-(4-methoxyphenyl) 3-methylbenzofuran-2-carboxamide, as protozoacides and bactericides for the control of coccidia in chickens. M. Descamps et al., Chimica Therapeutica, 1970, 5(3), 169-184, disclose N-(4-methoxyphenyl) and N-(4-ethoxyphenyl) benzofuran-2-carboxamides.

However there is a continuing need for new compounds useful as Orexin Receptor antagonists, especially those antagonists, which are selective OX1 receptor antagonists.

Thus, an object of the present invention is to provide a new class of compounds as Orexin Receptor antagonists, especially selective OX1 receptor antagonists, which may be effective in the treatment of Orexin Receptor related diseases.

Accordingly, the present invention provides compounds or a pharmaceutically acceptable salt thereof of formula (I) 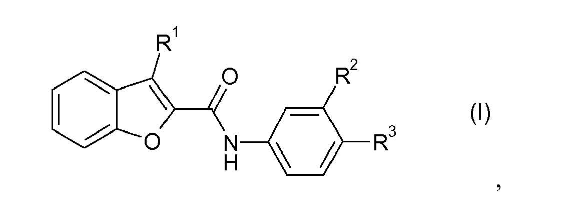 wherein
R¹ is H; CH₃; CH₂CH₃; OCH₃; NHC(O)CH₃; or CH₂OCH₃; preferably CH₃; CH₂CH₃; or OCH₃;
R² is H; or chloro; preferably H; and R³ is OC₁₋₄ alkyl; SC₁₋₄ alkyl; or N(C₁₋₄ alkyl)₂; preferably N(CH₂CH₃)₂;
or
R², R³ are joined together with the phenyl to which they are attached to form a heterobicycle T selected from the group consisting of dihydrobenzodioxin; dihydrobenzodioxepine; indole; benzoxazole; and benzothiazole, wherein T is optionally substituted with a methyl group,
for use in a method of treating or preventing diseases and disorders associated with the Orexin Receptors.

Preferred compounds are those selected from the group consisting of
N-(2,3-Dihydro-1,4-benzodioxin-6-yl)-3-methyl-2-benzofurancarboxamide;
N-[4-(Diethylamino)phenyl]-3-methyl-2-benzofurancarboxamide;
N-(2-Methyl-6-benzoxazolyl)-3-methyl-2-benzofurancarboxamide;
N-[4-(Diethylamino)phenyl]-3-(methoxymethyl)-2-benzofurancarboxamide;
N-[4-(Diethylamino)phenyl]-3-ethyl-2-benzofurancarboxamide;
N-(2-Methyl-6-benzoxazolyl)-3-ethyl-2-benzofurancarboxamide;
N-(3-Chloro-4-methoxyphenyl)-3-methyl-2-benzofurancarboxamide;
N-(3,4-dihydro-2H-1,5-benzodioxepin-7-yl)-3-methyl-2-benzofurancarboxamide;
N-(4-Ethoxyphenyl)-3-methyl-2-benzofurancarboxamide;
N-(1-Methyl-1*H-*indol-5-yl)-3-methyl-2-benzofurancarboxamide;
N-(4-Thioanisolyl)-3-methyl-2-benzofurancarboxamide;
N-[4-(Diethylamino)phenyl]-3-methoxy-2-benzofurancarboxamide;
N-[4-(Diethylamino)phenyl]-2-benzofurancarboxamide;
N-(2-Methyl-6-benzothiazolyl)-3-methyl-2-benzofurancarboxamide; and
N-[4-(Diethylamino)phenyl]-3-(acetylamino)-2-benzofurancarboxamide.

Furthermore, the present invention provides compounds or pharmaceutically acceptable salts thereof of formula (I) as defined above for use in a method of treating or preventing eating disorders, sleep disorders, cognitive dysfunctions in psychiatric and neurological disorders, drug dependence, obesity, or Type II Diabetes.

Preferred compounds of the present invention are novel as such.

Thus, the present invention also provides compounds or pharmaceutically acceptable salts thereof of formula (Ia), (Ib), or (Ic) 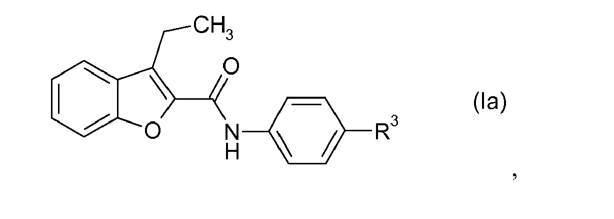 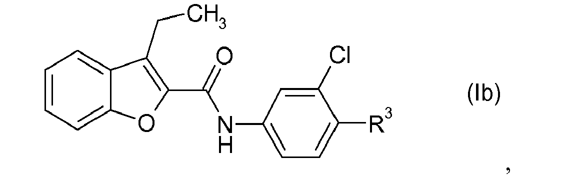 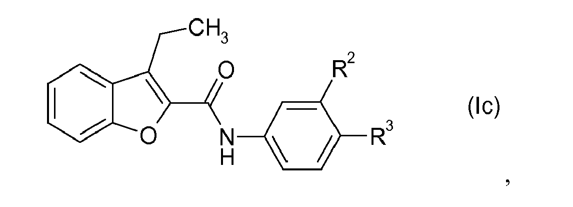 wherein in formulae (Ia), (Ib) R³ is OC₁₋₄ alkyl; SC₁₋₄ alkyl; or N(C₁₋₄ alkyl)₂; preferably N(CH₂CH₃)₂ and in formula (Ic) R², R³ are joined together with the phenyl to which they are attached to form a heterobicycle T selected from the group consisting of dihydrobenzodioxin; dihydrobenzodioxepine; indole; benzoxazole; and benzothiazole, wherein T is optionally substituted with a methyl group.

Preferred compounds are those selected from the group consisting of
N-(2-Methyl-6-benzoxazolyl)-3-methyl-2-benzofurancarboxamide;
N-[4-(Diethylamino)phenyl]-3-ethyl-2-benzofurancarboxamide;
N-(2-Methyl-6-benzoxazolyl)-3-ethyl-2-benzofurancarboxamide;
N-(1-Methyl-1*H*-indol-5-yl)-3-methyl-2-benzofurancarboxamide;
N-(4-Thioanisolyl)-3-methyl-2-benzofurancarboxamide;
N-[4-(Diethylamino)phenyl]-3-methoxy-2-benzofurancarboxamide;
N-(2-Methyl-6-benzothiazolyl)-3-methyl-2-benzofurancarboxamide; and
N-[4-(Diethylamino)phenyl]-3-(acetylamino)-2-benzofurancarboxamide.

In case a variable or substituent defined herein can be selected from a group of different variants and such variable or substituent occurs more than once the respective variants can be the same or different.

Within the meaning of the present invention the terms are used as follows:
"Alkyl" means a straight-chain or branched saturated hydrocarbon chain. "C₁₋₄ alkyl" means an alkyl chain having 1 - 4 carbon atoms, e.g. if present at the end of a molecule: methyl, ethyl, n-propyl, isopropyl, n-butyl, isobutyl, sec-butyl tert-butyl, or e.g. -CH₂-, -CH₂-CH₂-, -CH(CH₃)-, -CH₂-CH₂-CH₂-, -CH(C₂H₅)-, -C(CH₃)₂-, when two moieties of a molecule are linked by the alkyl group. Each hydrogen of a C₁₋₄ alkyl carbon may be replaced by a substituent as further specified.

Preferred compounds of formula (I) are those compounds in which one or more of the residues contained therein have the meanings given above, with all combinations of preferred substituent definitions being a subject of the present invention. With respect to all preferred compounds of the formula (I) the present invention also includes all tautomeric and stereoisomeric forms and mixtures thereof in all ratios, and their pharmaceutically acceptable salts as well as their isotopic derivatives.

In preferred embodiments of the present invention, the substituents R¹, R², R³ of formula (I) independently have the abovementioned meaning. Hence, one or more of the substituents R¹, R², R³ can have the preferred meanings given above. Compounds of the formula (I) in which some or all of the above-mentioned groups have the preferred meanings are also an object of the present invention. The same applies for compounds of formulae (Ia), (Ib) and (Ic), accordingly.

Where tautomerism, like e.g. keto-enol tautomerism, of compounds of formula (I) may occur, the individual forms, like e.g. the keto and enol form, are comprised separately and together as mixtures in any ratio. Same applies for stereoisomers, like e.g. enantiomers, cis/trans isomers, conformers and the like. The same applies for compounds of formulae (Ia), (Ib) and (Ic), accordingly.

Isotopic labeled compounds of formula (I) are also within the scope of the present invention. Methods for isotope labeling are known in the art. Preferred isotopes are those of the elements H, C, N, O and S. The same applies for compounds of formulae (Ia), (Ib) and (Ic), accordingly.

If desired, isomers can be separated by methods well known in the art, e.g. by liquid chromatography. Same applies for enantiomers by using e.g. chiral stationary phases. Additionally, enantiomers may be isolated by converting them into diastereomers, i.e. coupling with an enantiomerically pure auxiliary compound, subsequent separation of the resulting diastereomers and cleavage of the auxiliary residue. Alternatively, any enantiomer of a compound of formula (I) may be obtained from stereoselective synthesis using optically pure starting materials. The same applies for compounds of formulae (Ia), (Ib) and (Ic), accordingly.

In case the compounds according to formula (I) contain one or more acidic or basic groups, the invention also comprises their corresponding pharmaceutically or toxicologically acceptable salts, in particular their pharmaceutically utilizable salts. Thus, the compounds of the formula (I) which contain acidic groups can be used according to the invention, for example, as alkali metal salts, alkaline earth metal salts or as ammonium salts. More precise examples of such salts include sodium salts, potassium salts, calcium salts, magnesium salts or salts with ammonia or organic amines such as, for example, ethylamine, ethanolamine, triethanolamine or amino acids. Compounds of the formula (I) which contain one or more basic groups, i.e. groups which can be protonated, can be present and can be used according to the invention in the form of their addition salts with inorganic or organic acids. Examples for suitable acids include hydrogen chloride, hydrogen bromide, phosphoric acid, sulfuric acid, nitric acid, methanesulfonic acid, p-toluenesulfonic acid, naphthalenedisulfonic acids, oxalic acid, acetic acid, tartaric acid, lactic acid, salicylic acid, benzoic acid, formic acid, propionic acid, pivalic acid, diethylacetic acid, malonic acid, succinic acid, pimelic acid, fumaric acid, maleic acid, malic acid, sulfaminic acid, phenylpropionic acid, gluconic acid, ascorbic acid, isonicotinic acid, citric acid, adipic acid, and other acids known to the person skilled in the art. If the compounds of the formula (I) simultaneously contain acidic and basic groups in the molecule, the invention also includes, in addition to the salt forms mentioned, inner salts or betaines (zwitterions). The respective salts according to the formula (I) can be obtained by customary methods which are known to the person skilled in the art like, for example by contacting these with an organic or inorganic acid or base in a solvent or dispersant, or by anion exchange or cation exchange with other salts. The present invention also includes all salts of the compounds of the formula (I) which, owing to low physiological compatibility, are not directly suitable for use in pharmaceuticals but which can be used, for example, as intermediates for chemical reactions or for the preparation of pharmaceutically acceptable salts. The same applies for compounds of formulae (Ia), (Ib) and (Ic), accordingly.

The present invention provides compounds of general formula (I) as Orexin Receptor antagonists, preferably as selective OX1 receptor antagonists.

As described before, the orexins (orexin A or OX-A and orexin B or OX-B) are neuropeptides discovered in 1998 by two research groups; orexin A is a 33 amino acid peptide and orexin B is a 28 amino acid peptide which are derived from a common pre-propeptide (Sakurai et al., Cell, 1998, 92, 573-585; De Lecea L. et al, PNAS, 1998, 95, 322-27). Orexins are produced in the lateral hypothalamus and bind to two eponymous G-protein-coupled receptors (OX1 and OX2). The OX1 receptor is selective for OX-A, whereas the OX2 receptor binds both OX-A and OX-B.

The orexins stimulate food consumption in rats, suggesting a physiological role for these peptides as mediators of feeding behaviour (Sakurai T. et al, Cell, 1998, 92, 573-585; Ishii Y. et al, Behav.Brain.Res., 2005, vol. 157, 331-341; Thorpe A.J. et al, Brain Res., 2005, vol. 1050, 156-162).

The orexins regulate sleep-wake behaviour, indicating a potential therapeutic approach to narcolepsy, insomnia and other sleep disorders (Chemelli R.M. et al, Cell, 1999, 98, 437-451; Smith M.I. et al, Neurosci. Letters, 2003, 256-258; Midea M. et al., PNAS, 2004, vol. 101 (13), 4649-4654; Adamantidis A.R. et al., Nature, 2007, 420-425).

A post-mortem study of human narcolepsy patients failed to detect orexin peptides in the cortex and pons; there was an 80-100% reduction in the number of neurons containing detectable prepro-orexin mRNA or orexin-like immunoreactivity in the hypothalamus. Orexin A was undetectable in the cerebrospinal fluid of narcolepsy patients. The vast majority of patients with narcolepsy show decreased orexin A levels in the cerebrospinal fluid. A low cerebrospinal fluid concentration of orexin A is now one of the diagnostic criteria for narcolepsy-cataplexy (Sakurai T., Nature Reviews Neuroscience, 2007, 8, 171-181). Thus, blockade of Orexin receptors is expected to be of potential use in the treatment of insomnia.

It has been shown that animals pretreated with a selective OX1 antagonist (SB-334867) before naloxone-precipitated withdrawal demonstrated a striking reduction in several withdrawal symptoms (Sharf et al, Biol Psychiatry, 2008, 64(3), 175-183) Furthermore inhibition of orexin-1 receptors prevents yohimbine-induced reinstatement of ethanol and sucrose seeking in rats (Richards J.K. et al, Psychopharmacology, 2008, 199(1), 109-117) and blocks locomotor sensitization to cocaine in rats (Borgland S.L. et al, Neuron, 2006, 49, 589-601). The results indicate a critical role for orexin signaling in neuronal pathways leading to addiction and the potential use of antagonists in the treatment of drug dependence.

Unexpectedly, it has been found that benzofuran derivatives of formula (I) are antagonists of Orexin receptors, preferably selectively of the OX1 receptor. The present invention relates to the use of benzofuran-2-amide derivatives of formula (I) and pharmaceutically acceptable salts and solvates thereof in the treatment of disorders in which blockade of Orexin receptors is reasonably expected to be of therapeutic benefit e.g. eating disorders, sleep disorders, cognitive dysfunctions in psychiatric and neurological disorders, drug dependence, obesity and Type II Diabetes.

Accordingly, compounds of formula (I) may be used for the treatment of drug dependency including withdrawal symptoms from drugs of abuse such as alcohol, cocaine, opiates, nicotine, benzodiazepines and inhibition of tolerance induced by opioids. In addition, compounds of formula (I) and pharmaceutically acceptable salts and solvates thereof may be used to reduce withdrawal. Drug craving can be defined as the incentive motivation to self-administer a psychoactive substance that was previously consumed. Three main factors are involved in the development and maintenance of drug craving: (1) Dysphoric states during drug withdrawal can function as a negative reinforcer leading to craving; (2) Environmental stimuli associated with drug effects can become progressively more powerful (sensitization) in controlling drug seeking or craving, and (3) A cognition (memory) of the ability of drugs to promote pleasurable effects and to alleviate a dysphoric state during withdrawal.

The compounds of formula (I) may be used for the preparation of a medicament, and are suitable, for the prevention or treatment of diseases selected from the group consisting of eating disorders, sleep disorders, cognitive dysfunctions in psychiatric and neurological disorders, drug dependence, obesity, or Type II Diabetes.

Compounds of formula (I) are particularly suitable for use in the treatment of diseases or disorders selected from the group consisting of eating disorders, sleep disorders, cognitive dysfunctions in psychiatric and neurological disorders, drug dependence, obesity, or Type II Diabetes. Another aspect of the present invention is a method for the treatment or prophylaxis of diseases, which are related to the Orexin receptors such as eating disorders, sleep disorders, cognitive dysfunctions in psychiatric and neurological disorders, drug dependence, obesity, or Type II Diabetes comprising the administration to a patient a therapeutically effective amount of a compound of formula (I).

Accordingly, one aspect of the present invention is a compound or a pharmaceutically acceptable salt thereof of formulae (Ia), (Ib) or (Ic) as well as the individual compounds exemplified herein for use as a medicament. Those compounds can be used in a pharmaceutical composition comprising at least one of said compound or a pharmaceutically acceptable salt thereof together with a pharmaceutically acceptable carrier, optionally in combination with one or more other bioactive compounds or pharmaceutical compositions. Other bioactive compounds may be further compounds of the present invention, i.e. a mixture of two or more of these compounds. Further bioactive compounds are further Orexin 1 Receptor antagonists and the like.

Yet another aspect of the present invention is the use of a compound or a pharmaceutically acceptable salt thereof of the present invention for the manufacture of a medicament for the treatment or prophylaxis of diseases and disorders associated with the Orexin 1 Receptor.

Yet another aspect of the present invention is the use of a compound or a pharmaceutically acceptable salt thereof of the present invention for the manufacture of a medicament for the treatment or prophylaxis of eating disorders, sleep disorders, cognitive dysfunctions in psychiatric and neurological disorders, drug dependence, obesity, or Type II Diabetes. More specific diseases and disorders are mentioned above, which are also preferred for this aspect of the present invention.

Yet another aspect of the present invention is a method for treating, controlling, delaying or preventing in a mammalian patient in need of the treatment of one or more conditions selected from the group consisting of diseases and disorders associated with the Orexin 1 Receptor, wherein the method comprises the administration to said patient a therapeutically effective amount of a compound of the present invention or a pharmaceutically acceptable salt thereof.

Yet another aspect of the present invention is a method for treating, controlling, delaying or preventing in a mammalian patient in need of the treatment of one or more conditions selected from the group consisting of eating disorders, sleep disorders, cognitive dysfunctions in psychiatric and neurological disorders, drug dependence, obesity, and Type II Diabetes, wherein the method comprises the administration to said patient a therapeutically effective amount of a compound of the present invention or a pharmaceutically acceptable salt thereof. More specific diseases and disorders are mentioned above, which are also preferred for this aspect of the present invention.

"Pharmaceutical composition" means one or more active ingredients, and one or more inert ingredients that make up the carrier, as well as any product which results, directly or indirectly, from combination, complexation or aggregation of any two or more of the ingredients, or from dissociation of one or more of the ingredients, or from other types of reactions or interactions of one or more of the ingredients. Accordingly, the pharmaceutical compositions of the present invention encompass any composition made by admixing a compound of the present invention and a pharmaceutically acceptable carrier.

The active ingredients may be comprised in one or more different pharmaceutical compositions (combination of pharmaceutical compositions).

The term "pharmaceutically acceptable salts" refers to salts prepared from pharmaceutically acceptable non-toxic bases or acids, including inorganic bases or acids and organic bases or acids.

The compositions include compositions suitable for oral, rectal, topical, parenteral (including subcutaneous, intramuscular, and intravenous), ocular (ophthalmic), pulmonary (nasal or buccal inhalation), or nasal administration, although the most suitable route in any given case will depend on the nature and severity of the conditions being treated and on the nature of the active ingredient. They may be conveniently presented in unit dosage form and prepared by any of the methods well-known in the art of pharmacy.

In practical use, the compounds of formula (I) can be combined as the active ingredient in intimate admixture with a pharmaceutical carrier according to conventional pharmaceutical compounding techniques. The carrier may take a wide variety of forms depending on the form of preparation desired for administration, e.g., oral or parenteral (including intravenous). In preparing the compositions for oral dosage form, any of the usual pharmaceutical media may be employed, such as water, glycols, oils, alcohols, flavoring agents, preservatives, coloring agents and the like in the case of oral liquid preparations, such as, for example, suspensions, elixirs and solutions; or carriers such as starches, sugars, microcrystalline cellulose, diluents, granulating agents, lubricants, binders, disintegrating agents and the like in the case of oral solid preparations such as powders, hard and soft capsules and tablets, with the solid oral preparations being preferred over the liquid preparations.

Because of their ease of administration, tablets and capsules represent the most advantageous oral dosage unit form in which case solid pharmaceutical carriers are obviously employed. If desired, tablets may be coated by standard aqueous or nonaqueous techniques. Such compositions and preparations should contain at least 0.1 percent of active compound. The percentage of active compound in these compositions may, of course, be varied and may conveniently be between about 2 percent to about 60 percent of the weight of the unit. The amount of active compound in such therapeutically useful compositions is such that an effective dosage will be obtained. The active compounds can also be administered intranasally, for example, as liquid drops or spray.

The tablets, pills, capsules, and the like may also contain a binder such as gum tragacanth, acacia, corn starch or gelatin; excipients such as dicalcium phosphate; a disintegrating agent such as corn starch, potato starch, alginic acid; a lubricant such as magnesium stearate; and a sweetening agent such as sucrose, lactose or saccharin. When a dosage unit form is a capsule, it may contain, in addition to materials of the above type, a liquid carrier such as a fatty oil.

Various other materials may be present as coatings or to modify the physical form of the dosage unit. For instance, tablets may be coated with shellac, sugar or both. A syrup or elixir may contain, in addition to the active ingredient, sucrose as a sweetening agent, methyl and propylparabens as preservatives, a dye and a flavoring such as cherry or orange flavor.

Compounds of formula (I) may also be administered parenterally. Solutions or suspensions of these active compounds can be prepared in water suitably mixed with a surfactant such as hydroxypropyl-cellulose. Dispersions can also be prepared in glycerol, liquid polyethylene glycols and mixtures thereof in oils. Under ordinary conditions of storage and use, these preparations contain a preservative to prevent the growth of microorganisms.

The pharmaceutical forms suitable for injectable use include sterile aqueous solutions or dispersions and sterile powders for the extemporaneous preparation of sterile injectable solutions or dispersions. In all cases, the form should be sterile and should be fluid to the extent that easy syringability exists. It should be stable under the conditions of manufacture and storage and should be preserved against the contaminating action of microorganisms such as bacteria and fungi. The carrier can be a solvent or dispersion medium containing, for example, water, ethanol, polyol (e.g., glycerol, propylene glycol and liquid polyethylene glycol), suitable mixtures thereof, and vegetable oils.

Any suitable route of administration may be employed for providing a mammal, especially a human, with an effective dose of a compound of the present invention. For example, oral, rectal, topical, parenteral, ocular, pulmonary, nasal, and the like may be employed. Dosage forms include tablets, troches, dispersions, suspensions, solutions, capsules, creams, ointments, aerosols, and the like. Preferably compounds of formula (I) are administered orally.

The effective dosage of active ingredient employed may vary depending on the particular compound employed, the mode of administration, the condition being treated and the severity of the condition being treated. Such dosage may be ascertained readily by a person skilled in the art.

The same applies for compounds of formula (Ia), (Ib) and (Ic).

Starting materials for the synthesis of preferred embodiments of the invention as well as compounds of the present invention may be purchased from commercially available sources such as Array, Sigma Aldrich, Acros, Fisher, Fluka, ABCR or can be synthesized using known methods by one skilled in the art.

In general, several methods are applicable to prepare compounds of the present invention. In some cases various strategies can be combined. Sequential or convergent routes may be used.

Compounds of formula (I) may be prepared according to Scheme 1 below.

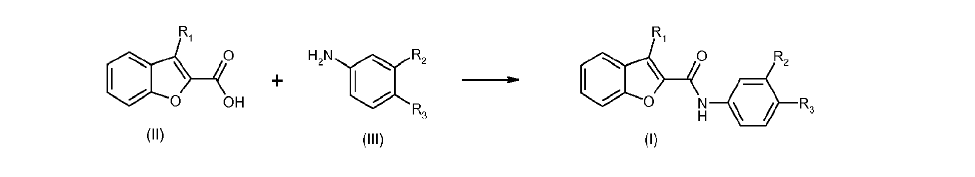

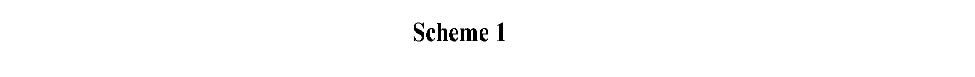

Amide coupling of acid (II) with amine (III) produces amide (I). Standard conditions for such couplings have been reviewed in Tetrahedron, 2005, 61(46), pp.10827-10852.

Accordingly, a further aspect of the present invention is a method of preparing a compound of formula (I) comprising the step of
- reacting a compound of formula (II) 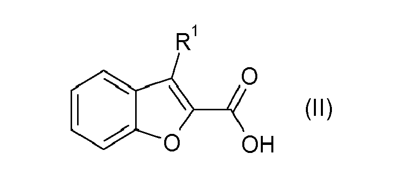 with a compound of formula (III) 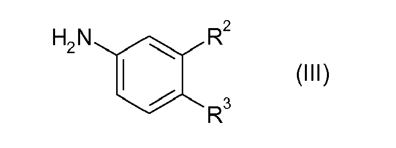 to yield a compound of formula (I).

The compounds of formula (II) where R¹ = CH₃ and R¹= -CH₂OCH₃ are commercially available. The compounds of formula (II) where R¹ = OCH₃ can be prepared according to Scheme 2 below:

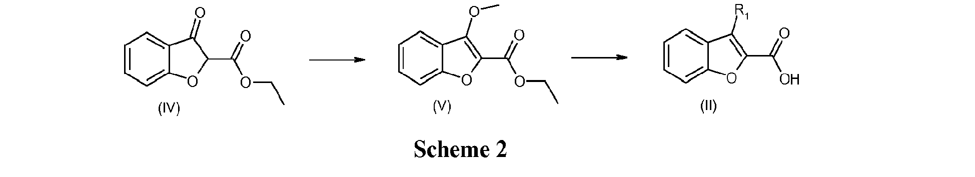

Alkylation of commercially-available ketone (IV) with e.g. methyl sulphate in the presence of a base such as potassium carbonate in a solvent such as acetone produces methyl ether (V), which can be hydrolysed using e.g. lithium hydroxide in tetrahydrofuran / water followed by acidification to give a compound of formula (II) where R¹ = OCH₃.

The compound of formula (II) where R¹=NHC(O)CH₃ can be prepared according to Scheme 3 below:

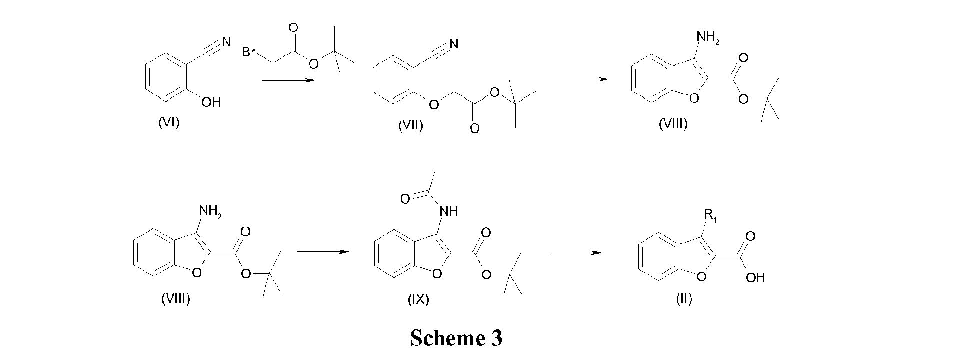

Alkylation of 2-cyanophenol (VI) with a halo-acetate derivative, e.g. tert-butyl bromoacetate in the presence of a base e.g. potassium carbonate in a solvent such as acetonitrile produces phenoxy-ester (VII), which can be cyclised to the 3-aminobenzofuran (VIII) by treatment with a strong base such as sodium hydride in a solvent such as dimethyl sulfoxide. Treatment of amine (VIII) with e.g. acetic anhydride affords acetamide (IX), which can be converted into the compound of formula (II) where R¹=NHC(O)CH₃ using e.g. trifluoroacetic acid in dichloromethane.

The compound of formula (II) where R¹=ethyl can be prepared according to Scheme 4 below:

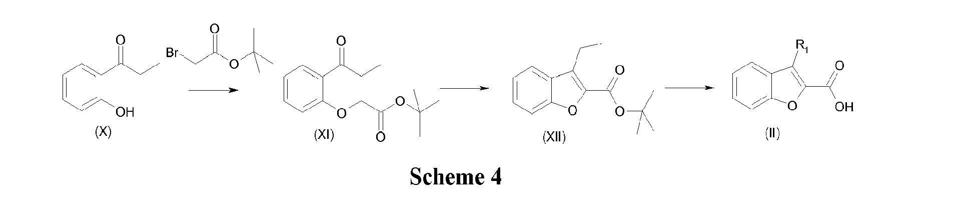

Alkylation of 2'-hydroxypropiophenone (X) with a halo-acetate derivative, e.g. tert-butyl bromoacetate in the presence of a base e.g. potassium carbonate in a solvent such as acetonitrile produces the keto-phenoxy-ester (XI). Treatment of (XI) with a base such as sodium hydride in a solvent such as DMSO, or with a base such as potassium carbonate in a solvent such as DMF at a temperature above 100 °C produces the 3-ethylbenzofuran-2-ester (XII), which can be converted into the compound of formula (II) where R¹=Ethyl using e.g. trifluoroacetic acid in dichloromethane.

### Examples

### Biological Assays

### Cell Lines

A CHO-K1 cell line stably expressing human Orexin-1 receptor (hOX1R, coding sequence of AF041243) was obtained from Euroscreen/PerkinElmer (CHO-hOX1R, ES-330-C).
A plasmid containing the human Orexin-2 receptor (hOX2R) cDNA was purchased from RZPD (RZPDo834A1045-pT-REx-DEST30). The human Orexin-2 receptor coding region was amplified in a gradient PCR reaction using hOX2Rfor (5' ccaggatccgc caccatgtccggcaccaaattggaggactcc) and hOX2Rrev (5' ccgcggccgcctaccagttttgaagtggtcctgc) primers containing restriction sites for the restriction enzymes BamHI and NotI. The PCR reaction was performed with the LightCycler system from Roche Applied Science. The amplified DNA fragment was digested with restriction enzymes BamHI and Notl (Fermentas) and subsequently cloned into the BamHI/NotI sites of the pFB-Neo vector (Stratagene) thereby generating plasmid pFB-Neo-hOX2R.
For a stably expressing CHO-hOX2R cell line viral transduction of CHO-K1 cells were performed by using the pFB-Neo-hOX2R plasmid and the pVPack vector system from Stratagene according to the manufacturer's manual. Cells expressing the hOX2 receptor were selected using G418 at a final concentration of 500µg/ml and single cell clones were produced by limited dilution cloning.

### Cell Culture

CHO-hOX1R cells were grown in HAM F12 Nutrient Mixture (Sigma, N6658), 10%Fetal Bovine Serum (Sigma, F9665), 1% Penstrep (Sigma, p4333) and 400mg/ml G418 (Sigma, A1720). CHO-hOX2R cells were grown in DMEM F12 medium (Sigma, D8437) supplemented with 10%Fetal Bovine Serum (Sigma, F9665), 1% Penstrep (Sigma, p4333) and 500mg/ml G418 (Sigma, A1720). Cells were maintained under 5% CO₂ atmosphere at 37°C. Cells were passaged every 2-3 days.

### Ca²⁺-flux Assay for EC50/IC50 Determination

CHO-hOX1R and CHO-hOX2R cells were seeded at a density of 7500 cells per well into black, 384 well Costar Cellbind plates and cultured overnight in the appropriate cell culture medium as described above without G418 selection. The next day the medium was removed and the cells were incubated for 1.5hrs at 37°C in fluo-4, AM dye solution [2µM fluo-4,AM (Molecular Probes; F-14202, Lot#28C1-12) in 5mM probenecid (Sigma, P-8761, Lot# 121K1662), 0.1% Bovine Serum Albumin (BSA, MERCK, 1.12018.0100), 1xHBSS (Invitrogen, 14025-050), 20mM HEPES (Invitrogen,. 15630-056)]. For IC50 determination of compounds the dye supernatant was removed and cells were incubated for 20min at 37°C in probenecid buffer [5mM probenecid, 0.1% BSA, 1xHBSS, 20mM HEPES, 1%DMSO (Merck, 1.02931.1000)] containing compounds at concentrations ranging from 1.28nM to 20µM (7 dilution steps at 1:5, last compound concentration is 0nM). All compound concentrations were measured in triplicates. Subsequently, agonist solution (5nM Orexin-A, TOCRIS, 1455) in 5mM probenecid, 0.1% BSA, 1xHBSS, 20mM HEPES was added during incubation of the plate at 37°C in the fluorescence plate reader (FlexStation, Molecular Devices). Fluorescence was measured for 0-60 seconds per well at an excitation of 485nm and emission of 538nm.
For EC50 determination of Orexin-A the cells were incubated as described above for 20min at 37°C in probenecid buffer (5mM probenecid, 0.1% BSA, 1xHBSS, 20mM HEPES, 1%DMSO) without compound solution. Agonist injection occurred in the fluorescence plate reader (FlexStation, Molecular Devices) at different concentrations ranging from 0-100nM in 5mM probenecid, 0.1% BSA, 1xHBSS, 20mM HEPES. Detection of fluorescence was as described above.

The activities of compounds of formula (I) are listed in Table 1 below:
+++ IC50 < 300nM
++ IC50 <1 µM
+ IC50 <3 µM
- activity not significantly different to control

**Table 1: OX1 antagonism of compounds of formula (I)**

| Example | 0X1 | 0X2 |
|---|---|---|
| 1 | + | - |
| 2 | +++ | - |
| 3 | +++ | - |
| 4 | ++ | - |
| 5 | +++ | - |
| 6 | +++ | - |
| 7 | + | - |
| 8 | + | - |
| 9 | + | - |
| 10 | ++ | - |
| 11 | ++ | - |
| 12 | +++ | - |
| 13 | ++ | - |
| 14 | ++ | - |
| 15 | + | - |

The invention is illustrated by the Examples described below.

In the procedures that follow, after each starting material, reference to a description is typically provided. This is provided merely for assistance to the skilled chemist. The starting material may not necessarily have been prepared from the batch referred to.

### NMR Methods

NMR Spectroscopy was determined using either a Bruker AVANCE 400 MHz NMR, a Bruker DPX 250 MHz NMR, a Bruker DPX 360 MHz NMR or a Bruker DRX 500 MHz NMR. Values are reported as shifts (in ppm), with zero corresponding to tetramethylsilane as an internal standard. Chemical shifts are reported in ppm ([delta]) using the residual solvent line as internal standard. Splitting patterns are designed as s, singlet; d, doublet; t, triplet; q, quartet; m, multiplet; b, broad. The NMR spectra were recorded at a temperature ranging from 25 to 90 C. When more than one conformer was detected the chemical shifts for the most abundant one is reported.

### Analytical HPLC-MS

### Method A

Column: Waters Atlantis dC18 (2.1 x100mm, 3um column)
Flow rate: 0.6 ml/min
Solvent A: 0.1% Formic acid / water
Solvent B: 0.1% Formic acid / acetonitrile
Injection Volume: 3 µl
Column temperature: 40°C
UV Detection wavelength: 215nm
Eluent: 0 mins to 5 mins, constant gradient from 95% solvent A + 5% solvent B to 100% solvent B; 5 mins to 5.4 mins, 100% solvent B; 5.4 mins to 5.42 mins, constant gradient from 100% solvent B to 95% solvent A + 5% solvent B; 5.42 mins to 7.00 mins, 95% solvent A + 5% solvent B

### Method B

Column: Waters Atlantis dC18 (2.1 x 30mm, 3um column)
Flow rate: 1 ml/min
Solvent A: 0.1% Formic acid / water
Solvent B: 0.1% Formic acid / acetonitrile
Injection volume: 3ul
UV Detection wavelength: 215nm
Eluent: 0 mins to 1.5 mins, constant gradient from 95% solvent A + 5% solvent B to 100% solvent B; 1.5 mins to 1.6 mins, 100% solvent B; 1.60 min to 1.61 mins, constant gradient from 100% solvent B to 95% solvent A + 5% solvent B; 1.61 mins to 2.00 min, 95% solvent A + 5% solvent B.

### MS detection using Waters LCT or LCT Premier, or ZQ or ZMD

UV detection using Waters 2996 photodiode array or Waters 2787 UV or Waters 2788 UV

### Preparative HPLC-MS

Column: Waters SunFire Prep C18 OBD (5um 19 x 100mm)
Flow rate: 26ml/min
Solvent A: 0.1% TFA / water
Solvent B: 0.1% TFA / acetonitrile
Injection Volume: 1000µl
Column Temperature: room temperature
Detection: Mass directed
Eluent: 0 mins to 1 minute, 90% solvent A + 10% solvent B; 1 minute to 7.5 mins, constant gradient from 90% solvent A + 10% solvent B to 100% solvent B; 7.5 mins to 9 mins, 100% solvent B; 9 mins to 9.1 mins, constant gradient from 100% solvent B to 90% solvent A + 10% solvent B; 9.1 mins to 10 mins, 90% solvent A + 10% solvent B.
Waters Micromass Platform LCZ single quadrupole mass spectrometer
Waters 600 solvent delivery module
Waters 515 ancillary pumps
Waters 2487 UV detector
Gilson 215 autosampler and fraction collector

### Compound Naming

All compounds are named either using ACD Labs 10.0 naming software (which conforms to IUPAC naming protocols) or by analogy to conventional nomenclature familiar to a skilled practitioner. Some compounds are isolated as TFA salts, which is not reflected by the chemical name. Within the meaning of the present invention the chemical name represents the compound in neutral form as well as its TFA salt or any other salt, especially pharmaceutically acceptable salt, if applicable.

Flash silica gel chromatography was carried out on silica gel 230-400 mesh or on pre-packed silica cartridges.

### List of Abbreviations

- br s: broad singlet
- Boc: *tert*-butoxycarbonyl
- CDCl₃: Chloroform-*d* deuterated chloroform
- d: doublet
- DCM: dichloromethane
- DIPEA: *N,N*-diisopropylethylamine
- DMF: *N*,*N*-dimethylformamide
- Eq.: equivalent
- EtOAc: ethyl acetate
- EtOH: ethanol
- FCC: flash column chromatography
- HC1: hydrochloric acid
- HOBt: 1-hydroxybenzotriazole
- HBTU: *o*-benzotriazol-1-yl-N,N,N',N'-tetramethyluronium tetrafluoroborate
- HPLC: high performance liquid chromatography
- K₂CO₃: potassium carbonate
- LCMS: liquid chromatography and mass spectrometry
- LiOH: lithium hydroxide
- MeOH: methanol
- MeOD: deuterated methanol
- Me₂SO₄: dimethyl sulphate
- MgSO₄: magnesium sulphate
- m: multiplet
- ml: millilitre
- mmol/M: millimole/molar
- MW: molecular weight
- NaOEt: sodium ethoxide
- Na₂CO₃: sodium carbonate
- NMR: nuclear magnetic resonance
- q: quartet
- Rt: retention time
- s: singlet
- t: triplet
- TFA: 2,2,2-trifluoroacetic acid
- THF: tetrahydrofuran
- TLC: thin layer chromatography

### Preparation 1: 2-[2-(1-Propionyl)phenoxy]-tert-butyl acetate

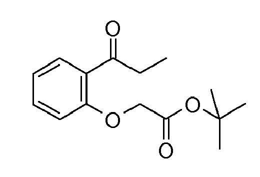

To a stirring solution of 2'-hydroxypropiophenone (0.2g, 0.18ml, 1.33mmol) and K₂CO₃ (0.37g, 2.66mmol) in acetonitrile was added *tert*-butyl bromoacetate (0.39g, 0.29ml, 2.00mmol). The resulting mixture was stirred at 80°C for 16 hours. Upon cooling to room temperature, the reaction mixture was filtered washing with additional acetonitrile. The filtrate was evaporated to dryness and purified by flash column chromatography on silica gel using 3:1 heptane / EtOAc as eluent to furnish the title compound as a yellow oil (134mg, 38% yield). ¹H NMR (400 MHz, CDCl₃) δ ppm 7.72 (1H, d, J=8.0 Hz, ArH), 7.42 (1H, t, J=8.0 Hz, ArH), 7.02 (1H, t, J=8.0 Hz, ArH), 6.81 (1H, d, J=8.0 Hz, ArH), 4.61 (2H, s, -CH₂), 3.13 (2H, q, J=8.0 Hz, -CH₂), 1.50 (9H, s, -O(CH₃)₃), 1.19 (3H, t, J=8.0 Hz, -CH₃).

### Preparation 2: 3-Ethylbenzofuran-2-carboxylic acid

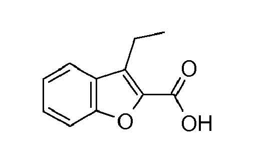

To a stirred solution of 2-[2-(1-propionyl)phenoxy]-*tert*-butyl acetate (Preparation 1, 134mg, 0.507mmol) in ethanol (2ml) was added a solution of sodium ethoxide (52mg, 0.760mmol) in ethanol (0.29ml). The reaction mixture was heated at 80°C for 16 hours. Upon cooling to room temperature the reaction mixture was evaporated to dryness and purified by flash column chromatography on silica gel using 2:1 heptane / EtOAc as eluent to furnish the title compound as a white solid (63mg, 68%). ¹H NMR (400 MHz, MeOD) δ ppm 7.58 (1H, d, J=8.0 Hz, ArH), 7.44 (1H, d, J=8.0 Hz, ArH), 7.30 (1H, t, J=8.0 Hz, ArH), 7.19 (1H, t, J=8.0 Hz, ArH), 3.10 (2H, q, J=8.0 Hz, -CH₂), 1.23 (3H, t, J=8.0 Hz, -CH₃).

### Preparation 3: Methyl 3-methoxybenzofuran-2-carboxylate

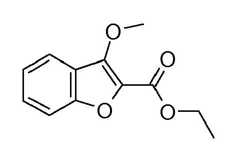

To a stirred solution of 2-ethoxycarbonyl-3-coumarone (200mg, 0.97mmol) in acetone (5ml) were added potassium carbonate (0.48g, 3.49mmol) and Me₂SO₄ (0.12m1s, 1.26mmol). The mixture was heated at 60°C for 6 hours. Upon cooling the reaction mixture was filtered, washing with additional acetone. The filtrate was evaporated to dryness and purified by flash column chromatography on silica gel using 5:1 heptane / EtOAc as eluent to furnish the title compound as an off-white solid (67mg, 31% yield). Method B HPLC-MS: MH⁺ requires m/z=221; Found: m/z=221, Rt=1.37 min (98%). ¹H NMR (250 MHz, CDCl₃) δ ppm 7.80 (1H, dt, J=2.5 7.5 Hz, ArH), 7.43-7.55 (2H, m, ArH), 7.30 (1H, dd, J=2.5 7.5 Hz, ArH), 4.46 (2H, q, J=7.5 Hz, -CH₂), 4.28 (3H, s, -OCH₃), 1.45 (3H, t, J=7.5 Hz, -CH₃).

### Preparation 4: 3-Methoxybenzofuran-2-carboxylic acid

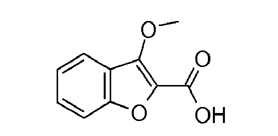

Methyl 3-methoxybenzofuran-2-carboxylate (Preparation 3, 65mg, 0.295mmol) was dissolved in a mixture of THF (3ml) and MeOH (2ml) and LiOH (2M solution in water, 1.80ml, 3.54mmol) was added. The solution was stirred at room temperature for 16 hours. The reaction mixture was acidified (pH 1-2, 1M HCl) and extracted into DCM (x3). The combined organics were dried (MgSO₄), filtered and concentrated to afford the title compound as an off-white solid (55mg, >95% yield) which was used in the next step without further purification. Method B HPLC-MS: MH⁺ requires m/z=193; Found: m/z=193, Rt = 1.09 min (95%). ¹H NMR (250 MHz, MeOD) δ ppm 7.85 (1H, dt, J=2.5 7.5 Hz, ArH), 7.48-7.51 (2H, m, ArH), 7.28-7.34 (1H, m, ArH), 4.24 (3H, s, -OCH₃).

### Preparation 5: tert-Butyl 2-(2-cyanophenoxy)acetate

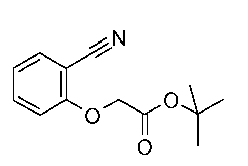

To a stirring solution of 2-cyanophenol (0.5g, 4.2mmol) and K₂CO₃ (2.32g, 16.8mmol) in acetone was added *tert*-butyl bromoacetate (0.98g, 0.73ml, 5.04mmol). The resulting mixture was stirred at 60°C for 16 hours. Upon cooling to room temperature, the reaction mixture was filtered, washing with additional acetone. The filtrate was evaporated to dryness to furnish the title compound as a clear yellow oil (980mg, 100% yield) which was used without further purification. Method B HPLC-MS: MH⁺ requires m/z=234; Found: m/z 234, Rt = 1.40 min (100%). ¹H NMR (500 MHz, CDCl₃) δ ppm 7.59 (1H, d, J=10.0 Hz, ArH), 7.52 (1H, dt, J=10.0 Hz, ArH), 7.05 (1H, t, J=10.0 Hz, ArH), 6.83 (1H, d, J=5.0 Hz, ArH), 4.67 (2H, s, - CH₂), 1.48 (9H, s, -O(CH₃)₃).

### Preparation 6: tert-Butyl 3-amino-benzofuran-2-carboxylate

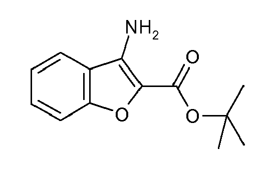

To a stirred solution of *tert*-butyl 2-(2-cyanophenoxy)acetate (0.98g, 4.2mmol) under nitrogen was added sodium hydride (60% dispersion in mineral oil, 189mg, 4.73mmol). The resulting suspension was stirred at 25°C for 16 hours. The reaction mixture was poured onto ice-water and extracted with diethyl ether (x3). The combined organics were washed with brine, dried (MgSO₄), filtered and concentrated to furnish the title compound as a clear yellow oil (630mg, 65% yield) which was used without further purification. Method B HPLC-MS: MH⁺ requires m/z=234; Found: m/z=234, Rt = 1.42 min (95%). ¹H NMR (250 MHz, CDCl₃) δ ppm 7.54 (1H, d, J=7.5 Hz, ArH), 7.43-7.46 (2H, m, ArH), 7.24 (1H, m, ArH), 4.88 (1H, br s, - NH₂), 1.66 (9H, s, -O(CH₃)₃).

### Preparation 7: tert-Butyl 3-(acetylamino)-2-benzofurancarboxylate

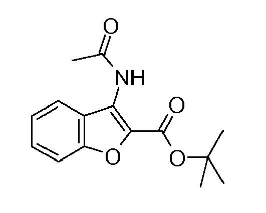

To a stirring solution of *tert*-butyl 3-amino-benzofuran-2-carboxylate (Preparation 6, 50mg, 0.214mmol) and DIPEA (0.075ml, 0.429mmol) in DCM under nitrogen was added acetyl chloride (0.042ml, 0.708mmol). The resulting suspension was stirred at 25°C for 7 days. 1N HCl was added and the organic layer washed with saturated aqueous NaHCO3 solution, dried (MgSO₄), filtered and concentrated to afford the crude product as an orange oil (60mg). Analysis of the ¹H NMR spectrum of the latter revealed a 2:1 mixture of the desired product and starting material, which was used without further purification. Method B HPLC-MS: MH⁺ requires m/z=276; Found: m/z 220 (MH⁺ - ^{t}Bu), Rt = 1.37 min (61%). ¹H NMR (250 MHz, CDCl₃) δ ppm 9.31 (1H, br s, -NH), 8.32 (1H, d, J=7.5 Hz, ArH), 7.41-7.44 (2H, m, ArH), 7.16-7.27 (1H, m, ArH), 2.27 (3H, s, -CH₃), 1.63 (9H, s, -O(CH₃)₃).

### Preparation 8: 3-(Acetylamino)-2-benzofurancarboxylic acid

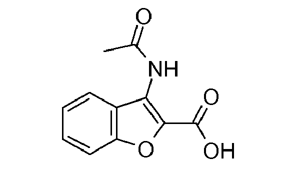

To a stirred solution of *tert*-butyl 3-(acetylamino)-2-benzofurancarboxylate (Preparation 7, 60mg, 2:1 mixture with Preparation 6) in DCM (2ml) was added TFA (1ml). The resulting solution was stirred at room temperature under nitrogen for 16 hours. The reaction mixture was evaporated to dryness, partitioned between DCM and water, the DCM layer dried (MgSO₄), filtered and concentrated to afford the crude product as an orange solid (33mg), which was taken onto the next step without further purification. Method B HPLC-MS: MH⁺ requires m/z=220; Found: m/z=220, Rt=0.97 min (56%).

### Example 1: N-(2,3-Dihydro-1,4-benzodioxin-6-yl)-3-methyl-2-benzofurancarboxamide

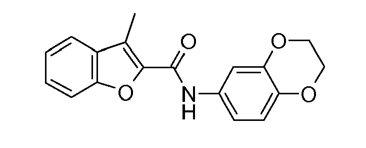

Purchased from Ambinter (50 Avenue de Versailles, Paris, France). Method A HPLC-MS: MH⁺ requires m/z=310; Found: m/z=310, Rt=4.57 min (91 %).

### Example 2: N-[4-(Diethylamino)phenyl]-3-methyl-2-benzofurancarboxamide

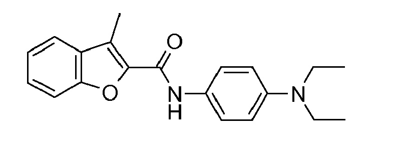

To a stirring solution of 3-methylbenzofuran-2-carboxylic acid (50mg, 0.284 mmol) in DMF (2ml) were added HBTU (215mg, 0.568mmol) and HOBt (84mg, 0.624mmol) sequentially. The resulting mixture was stirred at room temperature for 15 mins, after which time *N*,*N* diethyl-*p*-phenylenediamine (103mg, 0.624mmol) was added. The resulting solution was stirred at room temperature for 16 hours. The crude reaction mixture was evaporated to dryness and purified by preparative HPLC-MS to furnish the title compound as colourless oil (58.7 mg, 64% yield). Method A HPLC-MS: MH⁺ requires m/z=323; Found: m/z=323, Rt=3.22 min (100%). ¹H NMR (400 MHz, CDCl₃) δ ppm 8.74 (1H, br s, NH), 7.94 (2H, d, J=8.0 Hz, ArH), 7.94 (1H, d, J=8.0 Hz, ArH), 7.45-7.57 (4H, m, ArH), 7.33 (1H, t, J=8.0 Hz, ArH), 3.50-3.60 (4H, q, J=8.0 Hz, -(CH₂)₂), 2.66 (3H, s, -CH₃), 1.13 (6H, t, J=8.0 Hz, - (CH₃)₂).

### Example 3: N-(2-Methyl-6-benzoxazolyl)-3-methyl-2-benzofurancarboxamide

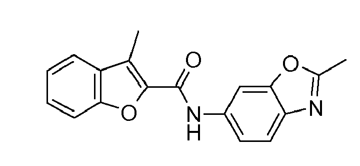

The title compound was prepared according to the method described for Example 2 using 3-methylbenzofuran-2-carboxylic acid (50mg, 0.284 mmol), HBTU (215mg, 0.568mmol), HOBt (84mg, 0.624mmol) and 2-methyl-1,3-benzoxazol-6-amine (93mg, 0.624mmol). The crude reaction mixture was evaporated to dryness and purified by preparative HPLC-MS to furnish the title compound as colourless oil (3.7mg, 4% yield). Method A HPLC-MS: MH⁺ requires m/z=307; Found: m/z 307, Rt = 4.49 min (95%). ¹H NMR (400 MHz, MeOD) δ ppm 8.21 (1H, s, ArH), 7.72 (1H, d, J=8.0 Hz, ArH), 7.56-7.62 (3H, m, ArH), 7.50 (1H, dt, J=4.0 8.0 Hz, ArH), 7.35 (1H, dt, J=4.0 8.0 Hz, ArH), 2.65 (3H, s, -CH₃), 2.63 (3H, s, -CH₃).

### Example 4: N-[4-(Diethylamino)phenyl]-3-(methoxymethyl)-2-benzofurancarboxamide

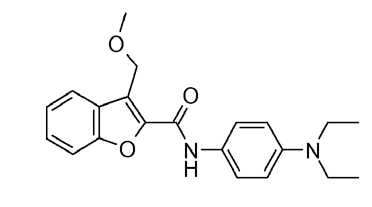

Purchased from Ambinter (50 Avenue de Versailles, Paris, France). Method A HPLC-MS: MH⁺ requires m/z=353; Found: m/z 353, Rt=3.19 min (96%).

### Example 5: N-[4-(Diethylamino)phenyl]-3-ethyl-2-benzofurancarboxamide

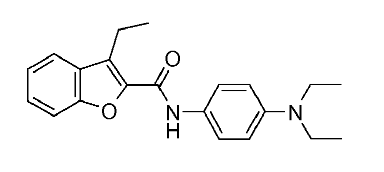

The title compound was prepared according to method described for Example 2 using 3-ethylbenzofuran-2-carboxylic acid (Preparation 2, 30mg, 0.16 mmol), HBTU (120mg, 0.32mmol), HOBt (47mg, 0.35mmol) and *N,N*-diethyl-*p*-phenylenediamine (57mg, 0.35mmol). Upon completion, the reaction was evaporated to dryness and purified by flash column chromatography on silica gel using 3:1 heptane / EtOAc as eluent to furnish the title compound as yellow-orange oil (9.2mg, 17% yield). Method A HPLC-MS: MH⁺ requires m/z=337; Found m/z=337, Rt = 3.50 min (99%). ¹H NMR (250 MHz, MeOD) δ ppm 7.72 (1H, d, J=7.5 Hz, ArH), 7.59 (1H, d, J=10.0 Hz, ArH), 7.43-7.49 (3H, m, ArH), 7.32 (1H, dt, J=2.5 7.5 Hz, ArH), 6.73 (2H, d, J=7.5 Hz, ArH), 2.65 (4H, q, J=7.5 Hz, -(CH₂)₂), 3.18 (2H, q, J=7.5 Hz, -CH₂), 1.32 (3H, t, J=7.5 Hz, -CH₃), 1.14 (6H, t, J=7.5 Hz, -(CH₃)₂).

### Example 6: N-(2-Methyl-6-benzoxazolyl)-3-ethyl-2-benzofurancarboxamide

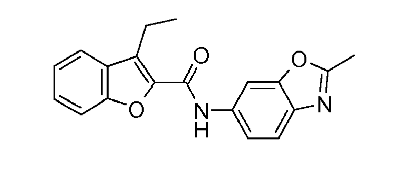

The title compound was prepared according to the method described for Example 2 using 3-ethylbenzofuran-2-carboxylic acid (Preparation 2, 30mg, 0.16 mmol), HBTU (120mg, 0.32mmol), HOBt (47mg, 0.35mmol) and 2-methyl-1,3-benzoxazol-6-amine (51mg, 0.35mmol). Upon completion, the reaction was evaporated to dryness and purified by flash column chromatography on silica gel using 3:1 heptane / EtOAc as eluent to furnish the title compound as yellow oil (6.5mg, 13% yield). Method A HPLC-MS: MH⁺ requires m/z=321; Found: m/z=321, Rt = 4.72 min (98%). ¹H NMR (250 MHz, CDCl₃) δ ppm 8.52 (1H, br s, - NH), 8.32 (1H, d, J=2.5 Hz, ArH), 7.71 (1H, d, J=10.0 Hz, ArH), 7.61 (1H, d, J=7.5 Hz, ArH), 7.54 (1H, d, J=7.5 Hz, ArH), 7.48 (1H, dt, J=2.5 7.5 Hz, ArH), 7.33 (1H, dt, J=2.5 7.5 Hz, ArH), 3.24 (2H, q, J=7.5 Hz, -CH₂), 2.65 (3H, s, -CH₃), 1.38 (3H, t, J=7.5 Hz, -CH₃).

### Example 7: N-(3-Chloro-4-methoxyphenyl)-3-methyl-2-benzofurancarboxamide

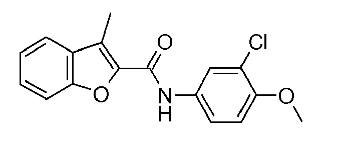

The title compound was prepared according to the method described for Example 2 using 3-methylbenzofuran-2-carboxylic acid (50mg, 0.284 mmol), HBTU (102mg, 0.270mmol), HOBt (42mg, 0.312mmol) and 3-chloro-*p*-anisidine (49mg, 0.312mmol). Upon completion, the reaction was evaporated to dryness and purified by preparative HPLC-MS to furnish the title compound (2mg, 4% yield). Method A HPLC-MS: MH⁺ requires m/z=316; Found: m/z=316, Rt = 4.83 min (99%). ¹H NMR (250 MHz, CDCl₃) δ ppm 8.27 (1H, br s, -NH), 7.72 (1H, d, J=2.5 Hz, ArH), 7.66 (1H, d, J=7.5 Hz, ArH), 7.59 (1H, dd, J=2.4 7.5 Hz, ArH), 7.44-7.53 (2H, m, ArH), 7.34 (1H, dt, J=2.5 7.5 Hz, ArH), 6.95 (1H, d, J=10.0 Hz, ArH), 3.93 (3H, s, -OCH₃), 2.69 (3H, s, -CH₃).

### Example 8: N-(3,4-dihydro-2H-1,5-benzodioxepin-7-yl)-3-methyl-2-benzofurancarboxamide

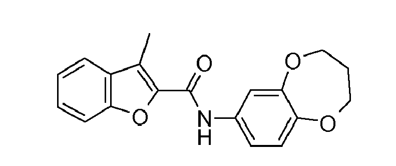

The title compound was prepared according to the method described for Example 2 using 3-methylbenzofuran-2-carboxylic acid (50mg, 0.284 mmol), HBTU (102mg, 0.270mmol), HOBt (42mg, 0.312mmol) and 3,4-dihydro-2*H*-1,5-benzodioxepin-7-amine (52mg, 0.312mmol). Upon completion, the reaction was evaporated to dryness and purified by preparative HPLC-MS to furnish the title compound (2.4mg, 5% yield). Method A HPLC-MS: MH⁺ requires m/z=324; Found: m/z=324, Rt = 4.61 min (100%). ¹H NMR (250 MHz, CDCl₃) δ ppm 8.25 (1H, br s, -NH), 7.65 (1H, d, J=7.5 Hz, ArH), 7.46-7.51 (2H, m, ArH), 7.41-7.43 (1H, m, ArH), 7.30-7.37 (1H, m, ArH), 7.23-7.24 (1H, m, ArH), 7.00 (1H, d, J=7.5 Hz, ArH), 4.18-4.26 (4H, m, -OCH₂CH₂CH₂O-) 2.69 (3H, s, -CH₃), 2.21 (2H, q, J=5.0 Hz, - OCH₂CH₂CH₂O-)

### Example 9: N-(4-Ethoxyphenyl)-3-methyl-2-benzofurancarboxamide

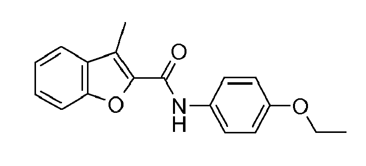

The title compound was prepared according to the method described for Example 2 using 3-methylbenzofuran-2-carboxylic acid (50mg, 0.284 mmol), HBTU (102mg, 0.270mmol), HOBt (42mg, 0.312mmol) and *p*-phenetidine (38mg, 0.312mmol). Upon completion, the reaction was evaporated to dryness and purified by preparative HPLC-MS to furnish the title compound (8.1mg, 20% yield). Method A HPLC-MS: MH⁺ requires m/z=296; Found: m/z=296, Rt = 4.75 min (100%). ¹H NMR (250 MHz, CDCl₃) δ ppm 8.27 (1H, br s, -NH), 7.58-7.67 (3H, m, ArH), 7.50-7.54 (1H, m, ArH), 7.43-7.49 (1H, m, ArH), 7.30-7.36 (1H, m, ArH), 6.89-6.95 (2H, m, ArH), 4.05 (2H, q, J=7.5 Hz, -OCH₂), 2.69 (3H, s, -CH₃), 1.43 (3H, t, J=7.5 Hz, -OCH₂CH₃).

### Example 10: N-(1-Methyl-1H-indol-5-yl)-3-methyl-2-benzofurancarboxamide

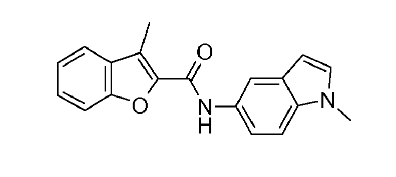

The title compound was prepared according to the method described for Example 2 using 3-methylbenzofuran-2-carboxylic acid (50mg, 0.284mmol), HBTU (102mg, 0.270mmol), HOBt (42mg, 0.312mmol) and 1-methyl-1*H-*indol-5-amine (46mg, 0.312mmol). Upon completion, the reaction was evaporated to dryness and purified by preparative HPLC. Analysis of the corresponding ¹H NMR spectrum revealed the presence of trace amounts of the starting material 3-methylbenzofuran-2-carboxylic acid. The residue following preparative HPLC-MS was reconstituted in EtOAc and washed with 1M Na₂CO₃, the organic layer was dried (MgSO₄), filtered and concentrated to furnish the title compound (10.3mg, 24% yield). Method A HPLC-MS: MH⁺ requires m/z=305; Found: m/z=305, Rt = 4.70 min (100%). ¹H NMR (250 MHz, CDCl₃) δ ppm 8.42 (1H, br s, -NH), 8.07 (1H, d, J=2.5 Hz, ArH), 7.65 (1H, d, J=7.5 Hz, ArH), 7.43-7.56 (3H, m, ArH), 7.30-7.37 (2H, m, ArH), 7.08 (1H, d, J=5.0 Hz, ArH), 6.49 (1H, dd, J=2.5 5.0 Hz, ArH), 3.91 (3H, s, -NCH₃), 2.72 (3H, s, -CH₃).

### Example 11: N-(4-Thioanisolyl)-3-methyl-2-benzofurancarboxamide

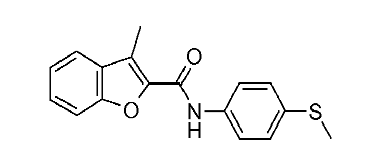

The title compound was prepared according to the method described for Example 2 using 3-methylbenzofuran-2-carboxylic acid (50mg, 0.284 mmol), HBTU (102mg, 0.270mmol), HOBt (42mg, 0.312mmol) and 4-(methylmercapto)aniline (43mg, 0.312mmol). Upon completion, the reaction was evaporated to dryness and purified by preparative HPLC-MS. Analysis of the corresponding ¹H NMR spectrum revealed the presence of trace amounts of the starting material 3-methylbenzofuran-2-carboxylic acid. The residue following preparative HPLC was reconstituted in EtOAc and washed with 1M Na₂CO₃, the organic layer was dried (MgSO₄), filtered and concentrated to furnish the title compound (11.9mg, 28% yield). Method A HPLC-MS: MH⁺ requires m/z=316; Found: m/z=316, Rt = 4.83 min (99%). ¹H NMR (250 MHz, CDCl₃) δ ppm 8.38 (1H, br s, -NH), 7.63-7.68 (3H, m, ArH), 7.44-7.54 (2H, m, ArH), 7.29-7.37 (3H, m, ArH), 2.69 (3H, s, -CH₃), 2.51 (3H, s, -SCH₃).

### Example 12: N-[4-(Diethylamino)phenyl]-3-methoxy-2-benzofurancarboxamide

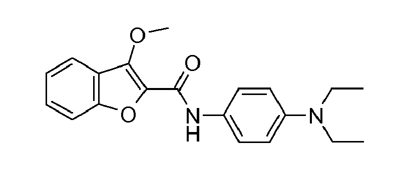

The title compound was prepared was prepared according to the method described for Example 2 using 3-methoxybenzofuran-2-carboxylic acid (Preparation 4, 55mg, 0.29mmol), HBTU (217mg, 0.57mmol), HOBt (85mg, 0.63mmol) and *N,N-*diethyl-*p*-phenylenediamine (0.105mls, 0.63mmol). Upon completion, the reaction was evaporated to dryness. A portion of the residue was purified by preparative HPLC-MS to furnish the title compound as a colourless oil (24.1mg). Method A HPLC-MS: MH⁺ requires m/z=339; Found: m/z=339, Rt = 3.13 min (95%). ¹H NMR (250 MHz, CDCl₃) δ ppm 8.91 (1H, br s, -NH), 7.87-7.97 (3H, m, ArH), 7.45-7.60 (4H, m, ArH), 7.31-7.38 (1H, m, ArH), 4.47 (3H, s, -OCH₃), 3.76 (4H, br, - (CH₂)₂), 1.22-1.27 (6H, m, -(CH₃)₂).

### Example 13: N-[4-(Diethylamino)phenyl]-2-benzofurancarboxamide

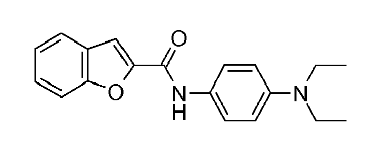

Purchased from Chembridge (16981 Via Tazon, San Diego, USA). Method A HPLC-MS: MH⁺ requires m/z=309; Found: m/z=309, Rt = 2.98 min (99%).

### Example 14: N-(2-Methyl-6-benzothiazolyl)-3-methyl-2-benzofurancarboxamide

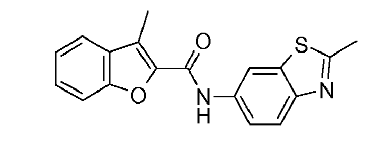

The title compound was prepared according to the method described for Example 2 using 3-methylbenzofuran-2-carboxylic acid (25mg, 0.142 mmol), HBTU (108mg, 0.284mmol), HOBt (42mg, 0.312mmol) and 2-methyl-1,3-benzothiazol-6-amine (51mg, 0.312mmol). Upon completion, the reaction was evaporated to dryness. The residue was purified by flash column chromatography on silica gel using 4:1 heptane / EtOAc as eluent to furnish the title compound as yellow oil (10.9mg, 24% yield). Method A HPLC-MS: MH⁺ requires m/z=323; Found: m/z=323, Rt = 4.67 min (100%). ¹H NMR (250 MHz, MeOD) δ ppm 8.47 (1H, d, J=2.5 Hz, ArH), 7.85 (1H, d, J=7.5 Hz, ArH), 7.71-7.77 (2H, m, ArH), 7.61 (1H, t, J=10.0 Hz, ArH), 7.50 (1H, dt, J=2.5 7.5 Hz, ArH), 7.35 (1H, dt, J=2.5 7.5 Hz, ArH), 2.82 (3H, s, -CH₃), 2.65 (3H, s, -CH₃).

### Example 15: N-[4-(Diethylamino)phenyl]- 3-(acetylamino)-2-benzofurancarboxamide

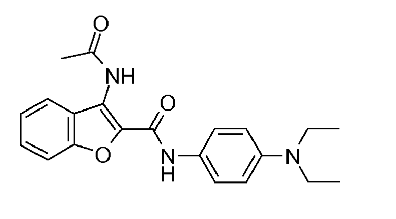

The title compound was prepared according to the method described for Example 2 using 3-(acetylamino)-2-benzofurancarboxylic acid (Preparation 8, 33mg, 0.15mmol), HBTU (114mg, 0.30mmol), HOBt (45mg, 0.33mmol) and *N*,*N-*diethyl-*p*-phenylenediamine (0.055mls, 0.33mmol). Upon completion, the reaction was evaporated to dryness and purified by flash column chromatography on silica gel using 4:1 heptane / EtOAc as eluent to furnish the title compound as yellow-orange oil (0.6mg). Method A HPLC-MS: MH⁺ requires m/z=366; Found: m/z 366, Rt = 3.05 min (100%). ¹H NMR (250 MHz, MeOD) δ ppm 7.05 (1H, d, J=7.5 Hz, ArH), 7.46-7.61 (4H, m, ArH), 7.31 (1H, t, J=7.5 Hz, ArH), 6.74 (2H, d, J=10.0 Hz, ArH), 3.38 (4H, q, J=7.5 Hz , -(CH₂)₂), 2.28 (3H, s, -COCH₃), 1.16 (6H, t, J=7.5 Hz, -(CH₃)₂).

## Claims

1. A compound or a pharmaceutically acceptable salt thereof of formula (I) 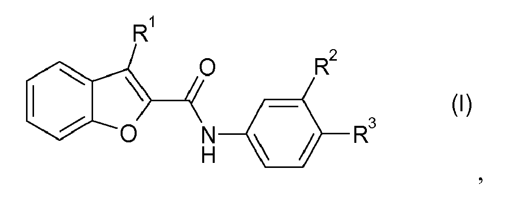 wherein
R¹ is H; CH₃; CH₂CH₃; OCH₃; NHC(O)CH₃; or CH₂OCH₃;
R² is H; or chloro; and R³ is OC₁₋₄ alkyl; SC₁₋₄ alkyl; or N(C₁₋₄ alkyl)₂;
or
R², R³ are joined together with the phenyl to which they are attached to form a heterobicycle T selected from the group consisting of dihydrobenzodioxin; dihydrobenzodioxepine; indole; benzoxazole; and benzothiazole, wherein T is optionally substituted with a methyl group,
for use in a method of treating or preventing diseases and disorders associated with the Orexin Receptors.

2. A compound or a pharmaceutically acceptable salt thereof of formula (I) as defined in claim 1 for use in a method of treating or preventing eating disorders, sleep disorders, cognitive dysfunctions in psychiatric and neurological disorders, drug dependence, obesity, or Type II Diabetes.

3. A compound of claim 1 or 2, wherein R¹ is CH₃; CH₂CH₃; or OCH₃.

4. A compound of any of claims 1 to 3, wherein R² is H.

5. A compound of any of claims 1 to 4, wherein R³ is N(CH₂CH₃)₂.

6. A compound of any of claims 1 to 5, wherein the compound is selected from the group consisting of
N-(2,3-Dihydro-1,4-benzodioxin-6-yl)-3-methyl-2-benzofurancarboxamide;
N-[4-(Diethylamino)phenyl]-3-methyl-2-benzofurancarboxamide;
N-(2-Methyl-6-benzoxazolyl)-3-methyl-2-benzofurancarboxamide;
N-[4-(Diethylamino)phenyl]-3-(methoxymethyl)-2-benzofurancarboxamide;
N-[4-(Diethylamino)phenyl]-3-ethyl-2-benzofurancarboxamide;
N-(2-Methyl-6-benzoxazolyl)-3-ethyl-2-benzofurancarboxamide;
N-(3-Chloro-4-methoxyphenyl)-3-methyl-2-benzofurancarboxamide;
N-(3,4-dihydro-2H-1,5-benzodioxepin-7-yl)-3-methyl-2-benzofurancarboxamide;
N-(4-Ethoxyphenyl)-3-methyl-2-benzofurancarboxamide;
N-(1-Methyl-1*H*-indo-1-5-yl)-3-methyl-2-benzofurancarboxamide;
N-(4-Thioanisolyl)-3-methyl-2-benzofurancarboxamide;
N-[4-(Diethylamino)phenyl]-3-methoxy-2-benzofurancarboxamide;
N-[4-(Diethylamino)phenyl]-2-benzofurancarboxamide;
N-(2-Methyl-6-benzothiazolyl)-3-methyl-2-benzofurancarboxamide; and
N-[4-(Diethylamino)phenyl]-3-(acetylamino)-2-benzofurancarboxamide.

7. A compound or a pharmaceutically acceptable salt thereof of formula (Ia), (Ib), or (Ic) 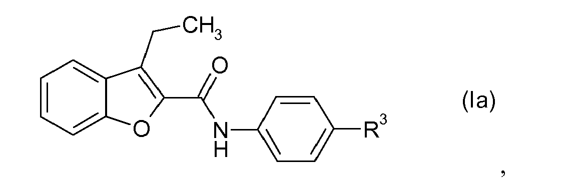 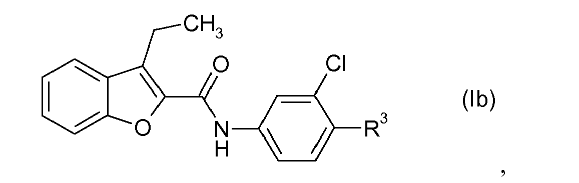 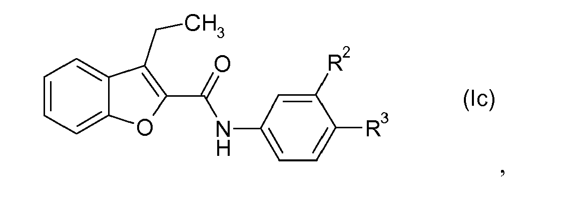 wherein in formulae (Ia), (Ib) R³ is OC₁₋₄ alkyl; SC₁₋₄ alkyl; or N(C₁₋₄ alkyl)₂; and in formula (Ic) R², R³ are joined together with the phenyl to which they are attached to form a heterobicycle T selected from the group consisting of dihydrobenzodioxin; dihydrobenzodioxepine; indole; benzoxazole; and benzothiazole, wherein T is optionally substituted with a methyl group.

8. A compound selected from the group consisting of
N-(2-Methyl-6-benzoxazolyl)-3-methyl-2-benzofurancarboxamide;
N-[4-(Diethylamino)phenyl]-3-ethyl-2-benzofurancarboxamide;
N-(2-Methyl-6-benzoxazolyl)-3-ethyl-2-benzofurancarboxamide;
N-(1-Methyl-1*H-*indol-5-yl)-3-methyl-2-benzofurancarboxamide;
N-(4-Thioanisolyl)-3-methyl-2-benzofurancarboxamide;
N-[4-(Diethylamino)phenyl]-3-methoxy-2-benzofurancarboxamide;
N-(2-Methyl-6-benzothiazolyl)-3-methyl-2-benzofurancarboxamide; and
N-[4-(Diethylamino)phenyl]-3-(acetylamino)-2-benzofurancarboxamide.

9. A pharmaceutical composition comprising at least one compound or a pharmaceutically acceptable salt thereof of claim 7 or 8 together with a pharmaceutically acceptable carrier, optionally in combination with one or more other bioactive compounds or pharmaceutical compositions.

10. A compound or a pharmaceutically acceptable salt thereof of claim 7 or 8 for use as a medicament.

11. A method of preparing a compound of formula (I) comprising the step of
- reacting a compound of formula (II) 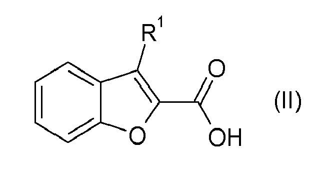 with a compound of formula (III) 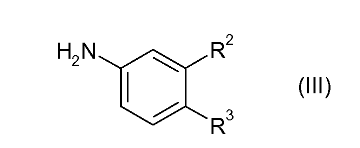 to yield a compound of formula (I).
